# EUROPÄISCHE PATENTSCHRIFT

(11) **EP 4 292 636 B1**
(45) Veröffentlichungstag und Bekanntmachung des Hinweises auf die Patenterteilung: **15.04.2026**
(21) Anmeldenummer: 23177239.3
(22) Anmeldetag: 05.06.2023
(51) Int. Cl.: A61M 25/00, A61M 27/00

(54) **IMPLANTAT ODER KATHETER**
IMPLANT OR CATHETER
IMPLANT OU CATHÉTER

(30) Priorität: 15.06.2022 DE 102022115028
(43) Veröffentlichungstag der Anmeldung: 20.12.2023
(73) Patentinhaber: Friedrich-Alexander-Universität Erlangen-Nürnberg, 91054 Erlangen (DE)
(72) Erfinder: BENKE, Elisabeth, 91052 Erlangen (DE); EBERT, Thomas, 90513 Zirndorf (DE); GRIGULL, Ronja-Celine, 71229 Leonberg (DE); PREIS, Alexander, 91052 Erlangen (DE); REITELSHÖFER, Sebastian, 91207 Lauf (DE); RIEKER, Ralf, 91054 Erlangen (DE)
(74) Vertreter: Dr. Gassner & Partner mbB

(56) Entgegenhaltungen:
- CN-A- 108 339 163
- DE-A1- 102010 008 070
- US-A- 5 645 528
- US-A1- 2008 208 083

## Beschreibung

Die Erfindung betrifft ein Implantat oder einen Katheter zum Einsetzen in die Harnröhre eines Patienten, mit einer Durchgangsöffnung zum Durchleiten von Urin, mit den Merkmalen des Oberbegriffs des Anspruchs 1.

Implantate dieser Art werden zum Beispiel bei Harninkontinenz verwendet. Sie weisen ein steuerbares Ventil auf, sodass die Abgabe von Urin vom Benutzer kontrolliert werden kann. Beispiele für derartige Implantate sind in den Druckschriften WO 2018/130358 A1 und WO 2019/243070 A1 beschrieben. Ein gattungsgemäßer Katheter ist aus der US 5 656 528 A bekannt. Die Druckschriften DE 10 2010 008070 A1, CN 108 339 163 A und US 2008/208083 A1 beschreiben ebenfalls medizinische Katheter.

Implantate zur Therapie von Harninkontinenz oder andere intraurethrale Implantate wie Stents sowie Urinkatheter weisen eine Durchgangsöffnung zum Durchleiten des Urinflusses auf. Allerdings wird die Zeitdauer, während ein derartiges Implantat oder ein derartiger Katheter in der Urethra eines Patienten verbleiben kann, unter anderem von dem Ausmaß der Bildung von Biofilmen und kristallinen Anlagerungen in der durchströmten Durchgangsöffnung begrenzt. Ein Austausch des Implantats oder Katheters ist üblicherweise nach einiger Zeit erforderlich, um eine Infektion des Patienten durch den Biofilm zu verhindern. Es ist daher wünschenswert, die Gebrauchsdauer eines derartigen Implantats oder Katheters zu verlängern, indem die Bildung eines Biofilms oder von Ablagerungen reduziert oder verhindert wird.

Der Erfindung liegt daher die Aufgabe zugrunde, ein Implantat oder einen Katheter zum Einsetzen in die Harnröhre eines Patienten anzugeben, dessen Gebrauchsdauer verlängert ist.

Zur Lösung dieser Aufgabe ist ein Implantat oder Katheter mit den Merkmalen des Anspruchs 1 vorgesehen.

Bei dem schlauchförmigen Implantat oder Katheter ist erfindungsgemäß ein aus einem Elastomer hergestellter, ein Lumen zum Durchleiten von Urin aufweisender Schlauch in der Durchgangsöffnung angeordnet, dessen Wandstärke so dünn ist, dass der Schlauch beim Durchströmen von Urin in Schwingungen versetzbar ist, wobei die Oszillation des Schlauchs ausschließlich durch den den Schlauch durchströmenden Urin erfolgt, wobei der Schlauch in dem Implantat oder dem Katheter unter Torsionsvorspannung befestigt ist, wobei ein sich auf der Oberfläche des Schlauchs befindender Biofilm durch die erzeugten Schwingungen lösbar und durch Urin wegspülbar ist. Bei dem Schlauch sind die geometrischen Parameter wie Wandstärke, Länge sowie der gewählte Werkstoff so aufeinander abgestimmt, dass sich beim Durchströmen von Urin die gewünschten Schwingungen einstellen, die die Entstehung eines Biofilms verringern oder vermeiden.

In dieser Anmeldung wird der Begriff "Lumen" für einen Hohlraum, d. h. einen durch Wände begrenzten Innenraum benutzt.

Die Erfindung beruht auf der Erkenntnis, dass ein Besatz mit einem Biofilm im Lumen eines Schlauchs, der sich in der Durchgangsöffnung eines Implantats oder eines Katheters befindet, verhindert oder wesentlich reduziert werden kann, indem ein Implantat mit einer vergleichsweise geringen Wandstärke verwendet wird, die bewirkt, dass das Implantat beim Durchströmen von Urin in Schwingungen versetzt wird, wodurch der Biofilm und gegebenenfalls vorhandene Anlagerungen weggespült werden. Durch diese mechanischen Schwingungen kann in vielen Fällen auch verhindert werden, dass überhaupt ein Biofilm entsteht. Die Oszillation des Implantats wird dabei ausschließlich durch den Urinfluss bewirkt, d. h. es ist kein elektrischer, mechanischer oder elektromechanischer Aktor erforderlich. Dementsprechend erfolgt die Reinigung des Implantats selbsttätig durch den den Schlauch durchströmenden Urin, ohne dass dazu eine externe Energiequelle benötigt wird. Das erfindungsgemäße Implantat bzw. der Katheter zeichnet sich dementsprechend durch einen besonders einfachen Aufbau aus und kann kostengünstig hergestellt werden.

Die Wirkung des erfindungsgemäßen Implantats oder Katheters beruht darauf, dass ein sich in dem Lumen des Schlauchs befindender Biofilm durch die Schwingungen gelöst und weggespült werden kann. Für die Funktion der Erfindung ist es daher wesentlich, dass die Wandstärke und andere geometrische Parameter des Schlauchs so gewählt sind, dass beim Durchströmen des Lumens des Schlauchs mit Urin Schwingungen erzeugt werden. Der Schlauch weist dazu eine besonders geringe Wandstärke auf und ist aus einem flexiblen, elastischen Material hergestellt.

Vorzugsweise ist zwischen der Außenseite des Schlauchs und der Innenseite der Durchgangsöffnung des Implantats oder Katheters zumindest abschnittsweise ein das Schwingen des Schlauchs ermöglichender radialer Freiraum vorhanden. Da der Schlauch von der Durchgangsöffnung des Implantats oder Katheters beabstandet ist, kann er in Radialrichtung schwingen.

Es hat sich als besonders günstig herausgestellt, dass die Wandstärke des Schlauchs 200 bis 600 µm beträgt, vorzugsweise 300 bis 500 µm und besonders bevorzugt etwa 400 µm. Bei einem Schlauch mit einer derartigen Wandstärke werden durch den strömenden Urin selbsttätig die erwünschten Schwingungen erzeugt, sodass der Biofilm von der Oberfläche des Schlauchs abgelöst und entfernt wird.

Im Hinblick auf die geometrischen Parameter wird es bevorzugt, dass der Innendurchmesser des Schlauchs 2,5 bis 3,5 mm beträgt. Vorzugsweise kann der Innendurchmesser etwa 3,0 mm betragen.

Eine Weiterbildung der Erfindung sieht vor, dass der in Schwingungen versetzbare Abschnitt ein mittlerer Abschnitt des Schlauchs ist, dessen Wandstärke im Vergleich zu Endabschnitten des Schlauchs verringert ist. Dementsprechend können Endabschnitte des Schlauchs eine größere Wandstärke bzw. Dicke aufweisen. Der mittlere Teil besitzt hingegen eine verringerte Wandstärke, sodass er beim Durchströmen von Urin zu Schwingungen angeregt wird. Alternativ kann der Schlauch jedoch auch eine konstante Wandstärke besitzen. Ein derartiger Schlauch kann besonders einfach hergestellt werden.

Gemäß der Erfindung ist der Schlauch in dem Implantat oder Katheter unter Torsionsvorspannung angebracht, indem der Schlauch mit einer zumindest geringfügigen Verdrehung um seine Längsachse befestigt ist. Diese Anbringung unter Torsion begünstigt die erwünschte Entstehung von Schwingungen des Schlauchs, wenn er durchströmt wird.

Eine alternative Variante der Erfindung sieht vor, dass der Schlauch in dem Implantat oder dem Katheter zwischen einer oberen Befestigungsposition und einer unteren Befestigungsposition implantiert ist, wobei die Länge des Implantats größer als der Abstand zwischen den beiden Befestigungspositionen ist. Es wurde herausgefunden, dass ein derartiges Implantat, das etwas länger als erforderlich ist, beim Durchströmen von Urin ebenfalls zu Schwingungen neigt, die die Reinigung des Lumens günstig beeinflussen. Der Schlauch kann eine konstante Wandstärke oder einen mittleren Abschnitt mit einer verringerten Wandstärke besitzen.

Eine weitere alternative Variante des Schlauchs des erfindungsgemäßen Implantats oder Katheters ist so geformt, dass Abschnitte mit vergrößertem Durchmesser sich mit Abschnitten mit verringertem Durchmesser abwechseln. Diese Ausführung weist in Längsrichtung eine wellenförmige Form auf. An jeder Position in Längsrichtung ist der Schlauch im Querschnitt kreisförmig, wobei der Durchmesser sich kontinuierlich verringert bzw. vergrößert. Versuche haben ergeben, dass auch diese Form des Schlauchs die Entstehung turbulenter Strömungen anregt, durch die gegebenenfalls vorhandene Biofilme oder Anlagerungen von der Oberfläche des Lumens gelöst und weggespült werden.

Der Schlauch des erfindungsgemäßen Implantats oder Katheters kann aus einem Elastomer hergestellt sein, besonders bevorzugt wird ein Silikonmaterial. Die Oberfläche kann mit einer antibakteriellen Beschichtung versehen sein.

Die Erfindung wird nachfolgend unter Bezugnahme auf die Zeichnungen erläutert. Die Zeichnungen sind schematische Darstellungen und zeigen:
- Fig. 1: ein erstes Ausführungsbeispiel eines erfindungsgemäßen Implantats;
- Fig. 2: das in Fig. 1 gezeigte Implantat im durchströmten Zustand;
- Fig. 3: ein zweites Ausführungsbeispiel eines erfindungsgemäßen Implantats;
- Fig. 4: das in Fig. 3 gezeigte Implantat im durchströmten Zustand;
- Fig. 5: ein drittes Ausführungsbeispiel eines erfindungsgemäßen Implantats; und
- Fig. 6: das in Fig. 5 gezeigte Implantat im durchströmten Zustand.

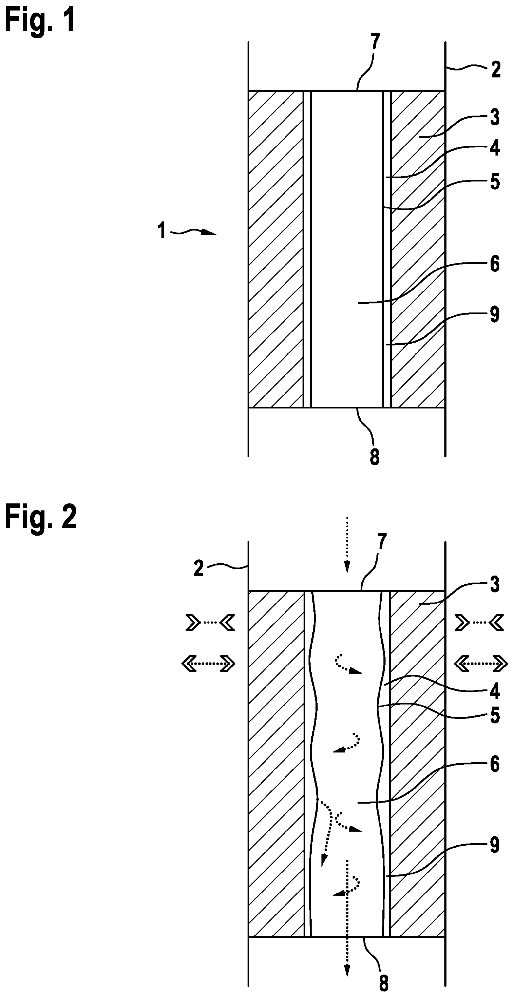

Fig. 1 zeigt ein Implantat 1, das gemäß einem ersten Ausführungsbeispiel im Inneren einer Harnröhre 2 eines Patienten befestigt ist. Das Implantat 1 besitzt einen rohrförmigen Grundkörper 3, der eine Durchgangsöffnung 4 aufweist, in der ein aus einem Elastomer hergestellter Schlauch 5 angeordnet ist, der ein Lumen 6 zum Durchleiten von Urin besitzt. Der Schlauch 5 ist an beiden Enden 7, 8 so an dem Grundkörper 3 des Implantats 1 befestigt, dass Urin ausschließlich durch das Lumen 6 fließen kann. Zwischen der Außenseite des das Lumen 6 aufweisenden Schlauchs 5 und der Innenseite des Grundkörpers 3 des Implantats befindet sich ein zylinderförmiger Freiraum 9. Die geometrischen Parameter des Schlauchs 5, d. h. die Wandstärke und seine Länge sind so gewählt, dass der Schlauch 5 beim Durchströmen von Urin in Schwingungen versetzt wird. Diese Schwingungen verhindern, dass sich im Inneren des Schlauchs 5 Ablagerungen wie ein Biofilm bilden. Falls bereits ein Biofilm entstanden ist, kann dieser durch die Bewegung des Schlauchs 5 gelöst und durch die strömende Flüssigkeit weggespült werden.

Der das Lumen 6 aufweisende Schlauch 5 ist zumindest geringfügig um seine Längsachse verdreht, d. h. tordiert, in der Durchgangsöffnung 4 des Grundkörpers 3 des Implantats 1 angeordnet. Dadurch wird eine geringe Torsionsvorspannung erzeugt, die die Entstehung von Schwingungen des Schlauchs 5 erleichtert.

Fig. 2 zeigt das in Fig. 1 gezeigte Implantat 1 im durchströmten Zustand. Wenn das Lumen 6 des in dem Grundkörper 3 aufgenommenen Schlauchs 5 des Implantats 1 von Urin durchströmt wird, werden Schwingungen bzw. Wellenbewegungen des Schlauchs 5 erzeugt, sodass ein beliebiger Punkt auf der Oberfläche des Schlauchs 5 radial nach außen und innen bewegt wird. Versuche haben ergeben, dass diese Wellenbewegungen mit einer turbulenten Strömung der durchströmenden Flüssigkeit einhergehen. Die mechanischen Schwingungen des Schlauchs 5 bewirken andererseits, dass Anlagerungen und Biofilme an der Oberfläche des Lumens 3 abgelöst und weggespült werden. Auf diese Weise wird die Innenseite des Schlauchs 5 des Implantats 1 gereinigt. Da das Wachstum von Biofilmen stark verringert wird, kann das Implantat 1 mit dem darin angeordneten Schlauch 5 wesentlich länger benutzt werden, bevor es aufgrund eines entstandenen Biofilms entfernt werden muss.

In dem dargestellten Ausführungsbeispiel beträgt die Wandstärke des schlauchförmigen Schlauchs 5 400 µm, der Innendurchmesser des Lumens 6 des Schlauchs 5 beträgt 3,0 mm.

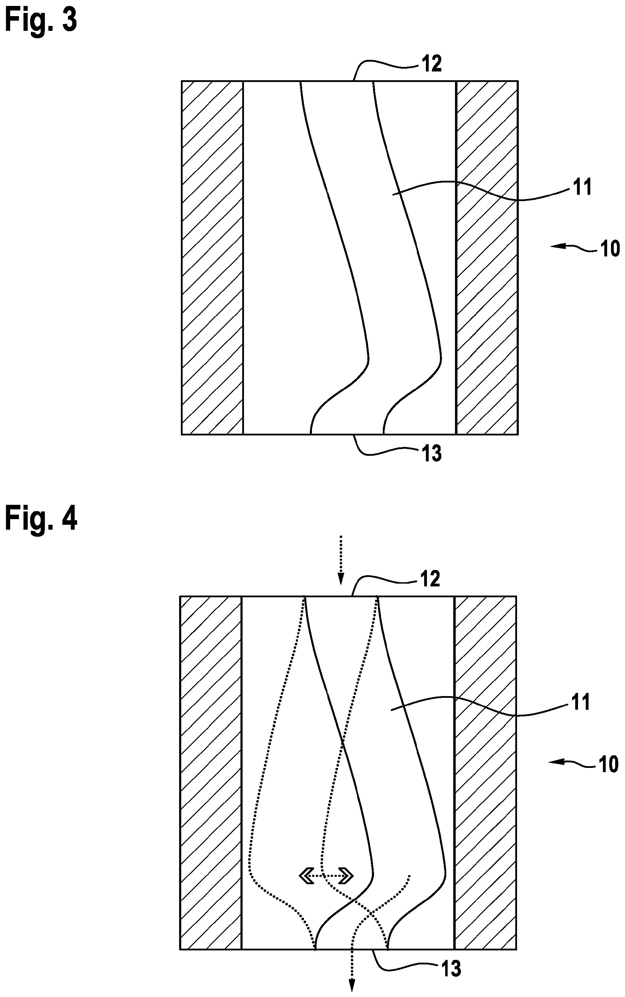

Fig. 3 ist ein zweites Ausführungsbeispiel und zeigt ein in der Harnröhre eines Patienten eingesetzten Implantats 10 mit einem Schlauch 11, der an einer oberen Position 12 und einer unteren Position 13 befestigt ist. Die Länge des Schlauchs 11 ist größer als der Abstand zwischen der oberen Position 12 und der unteren Position 13, sodass der Schlauch 11 im Ruhezustand, wenn er nicht von Urin durchströmt wird, schlaff ist.

Fig. 4 zeigt das Implantat 10 im durchströmten Zustand. Der Schlauch 11 des Implantats 10 wird unter der Wirkung des strömenden Urins in Schwingungen versetzt, die eine Ablösung von Biofilmen oder Anlagerungen bewirken. Voraussetzung dafür ist, dass der Schlauch 11 länger als der Abstand zwischen der oberen Position 12 und der unteren Position 13 ist. Dementsprechend erfolgt auch bei dem Implantat 10 eine selbsttätige Reinigung des Schlauchs 11.

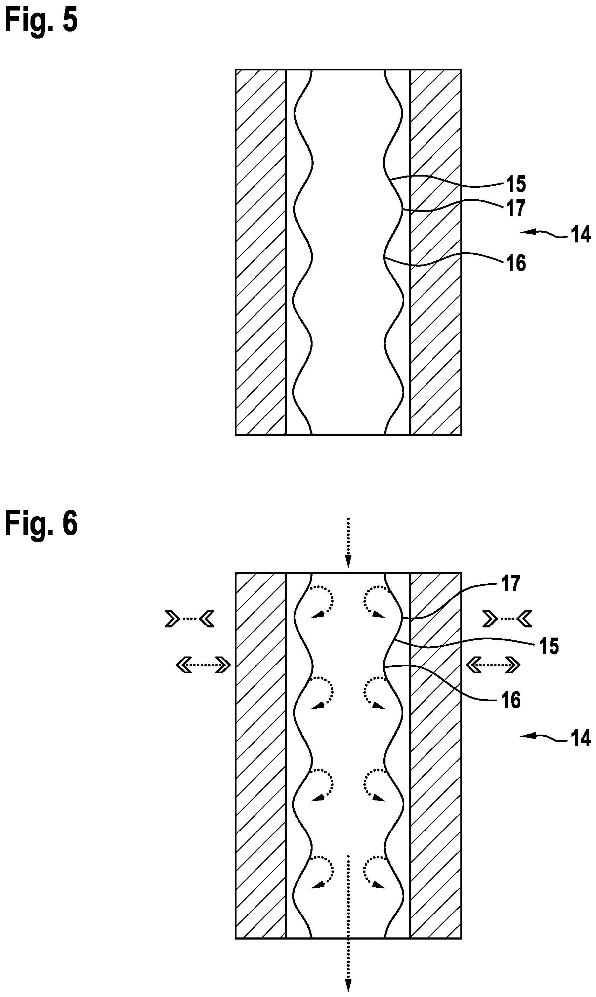

Fig. 5 ist ein weiteres Ausführungsbeispiel und zeigt ein Implantat 14, in dessen Durchgangsöffnung ein Schlauch 15 angeordnet ist, der so geformt ist, dass Abschnitte 17 mit vergrößertem Durchmesser sich mit Abschnitten 16 mit verringertem Durchmesser abwechseln. Das Implantat 14 weist somit im Ruhezustand, d. h. wenn es nicht von Urin durchflossen wird, eine wellenförmige Außenkontur auf. In einer zur Längsrichtung des Implantats 14 senkrecht geschnittenen Ebene wird die Außenkontur des Implantats 14 durch Kreise begrenzt, deren Durchmesser sich in Radialrichtung ändern, wenn Urin durch das Lumen des Schlauchs 15 des Implantats 14 strömt.

Fig. 6 zeigt, dass das Implantat 14 zu Schwingungen angeregt wird, wenn es durchströmt wird. Die Strömung im Inneren des Schlauchs 15 ist turbulent, wodurch auf das den Schlauch 15 wirkende Kräfte entstehen, die die Ablösung von Biofilmen und die gewünschte Reinigungswirkung bewirken.

### Bezugszeichen

- 1: Implantat
- 2: Harnröhre
- 3: Grundkörper
- 4: Durchgangsöffnung
- 5: Schlauch
- 6: Lumen
- 7: Ende
- 8: Ende
- 9: Freiraum
- 10: Implantat
- 11: Schlauch
- 12: obere Position
- 13: untere Position
- 14: Implantat
- 15: Schlauch
- 16: Abschnitt
- 17: Abschnitt

## Patentansprüche

1. Implantat (1, 10, 14) oder Katheter zum Einsetzen in die Harnröhre (2) eines Patienten, mit einer Durchgangsöffnung (4) zum Durchleiten von Urin, wobei ein aus einem Elastomer hergestellter, ein Lumen (6) zum Durchleiten von Urin aufweisender Schlauch (5, 11, 15) in der Durchgangsöffnung (4) angeordnet ist, dessen Wandstärke so dünn ist, dass der Schlauch (5, 11, 15) beim Durchströmen von Urin in Schwingungen versetzbar ist, **dadurch gekennzeichnet, dass** die Oszillation des Schlauchs (5, 11, 15) ausschließlich durch den den Schlauch (5, 11, 15) durchströmenden Urin erfolgt, wobei der Schlauch (5, 11, 15) in dem Implantat (1, 10, 14) oder dem Katheter unter Torsionsvorspannung befestigt ist, wobei ein sich auf der Oberfläche des Schlauchs (5, 11, 15) befindender Biofilm durch die erzeugten Schwingungen lösbar und durch Urin wegspülbar ist.

2. Implantat oder Katheter nach Anspruch 1, wobei zwischen der Außenseite des Schlauchs (5, 11, 15) und der Innenseite der Durchgangsöffnung (4) zumindest abschnittsweise ein das Schwingen des Schlauchs (5, 11, 15) ermöglichender zylinderförmiger Freiraum (9) vorhanden ist.

3. Implantat oder Katheter nach Anspruch 1 oder 2, wobei die Wandstärke des Schlauchs (5, 11, 15) 200 bis 600 µm beträgt, vorzugsweise 300 bis 500 µm und besonders bevorzugt etwa 400 µm.

4. Implantat oder Katheter nach einem der vorangehenden Ansprüche, wobei der Innendurchmesser des Lumens (6) des Schlauchs (5, 11, 15) 2,5 bis 3,5 mm und vorzugsweise etwa 3,0 mm beträgt.

5. Implantat oder Katheter nach einem der vorangehenden Ansprüche, wobei der in Schwingungen versetzbare Abschnitt des Schlauchs (5, 11, 15) ein mittlerer Abschnitt ist, dessen Wandstärke im Vergleich zu Endabschnitten des Schlauchs (5, 11, 15) verringert ist.

6. Implantat oder Katheter nach einem der vorangehenden Ansprüche, wobei der Schlauch (5, 11, 15) an einer oberen Position (12) und einer unteren Position (13) in dem Implantat (1, 10, 14) oder dem Katheter befestigt ist und die Länge des Schlauchs (5, 11, 15) größer als der Abstand zwischen der oberen Position (12) und der unteren Position (13) ist.

7. Implantat oder Katheter nach einem der vorangehenden Ansprüche, wobei der Schlauch (5, 11, 15) so geformt ist, dass Abschnitte (17) mit vergrößertem Durchmesser sich mit Abschnitten (16) mit verringertem Durchmesser abwechseln.

8. Implantat oder Katheter nach einem der vorangehenden Ansprüche, wobei der Schlauch (5, 11, 15) aus einem Elastomer, vorzugsweise aus einem Silikonmaterial, hergestellt ist.

## Claims

1. Implant (1, 10, 14) or catheter for insertion into the urethra (2) of a patient, with a passage opening (4) for passing urine, wherein a tube (5, 11, 15) made of an elastomer and having a lumen (6) for passing urine is arranged in the passage opening (4), the wall thickness of which tube is so thin that the tube (5, 11, 15) is not obstructed when urine flows through it, **characterized in that** the oscillation of the tube (5, 11, 15) is caused exclusively by the urine flowing through the tube (5, 11, 15), wherein the tube (5, 11, 15) is fixed in the implant (1, 10, 14) or the catheter under torsional preload, whereby a biofilm located on the surface of the tube (5, 11, 15) can be loosened by the vibrations generated and washed away by urine.

2. Implant or catheter according to claim 1, wherein a cylindrical free space (9) enabling the tube (5, 11, 15) to vibrate is provided at least in sections between the outside of the tube (5, 11, 15) and the inside of the passage opening (4).

3. Implant or catheter according to claim 1 or 2, wherein the wall thickness of the tube (5, 11, 15) is 200 to 600 µm, preferably 300 to 500 µm, and particularly preferably about 400 µm.

4. Implant or catheter according to one of the preceding claims, wherein the inner diameter of the lumen (6) of the tube (5, 11, 15) is 2.5 to 3.5 mm and preferably about 3.0 mm.

5. Implant or catheter according to one of the preceding claims, wherein the section of the tube (5, 11, 15) that can be set into vibration is a middle section whose wall thickness is reduced in comparison to end sections of the tube (5, 11, 15).

6. Implant or catheter according to any of the preceding claims, wherein the tube (5, 11, 15) is attached at an upper position (12) and a lower position (13) in the implant (1, 10, 14) or the catheter, and the length of the tube (5, 11, 15) is greater than the distance between the upper position (12) and the lower position (13).

7. Implant or catheter according to any of the preceding claims, wherein the tube (5, 11, 15) is shaped such that sections (17) of increased diameter alternate with sections (16) of decreased diameter.

8. Implant or catheter according to one of the preceding claims, wherein the tube (5, 11, 15) is made of an elastomer, preferably a silicone material.

## Revendications

1. Implant (1, 10, 14) ou cathéter destiné à être introduit dans l'urètre (2) d'un patient, ayant une ouverture de passage (4) pour le passage de l'urine, en ce qu'un tube (5, 11, 15) réalisé en un élastomère, présentant une lumière (6) pour le passage de l'urine est disposé dans l'ouverture de passage (4), dont l'épaisseur de paroi est si mince que le tube (5, 11, 15) peut être mis en vibration lors du passage de l'urine, **caractérisé en ce que** l'oscillation du tube (5, 11, 15) se produit exclusivement sous l'effet de l'urine passant à travers le tube (5, 11, 15), **en ce que** le tube (5, 11, 15) est fixé dans l'implant (1, 10, 14) ou le cathéter sous précontrainte de torsion, **en ce qu'**un biofilm se trouvant sur la surface du tube (5, 11, 15) peut être désolidarisé par les vibrations produites et éliminé par rinçage par l'urine.

2. Implant ou cathéter selon la revendication 1, en ce qu'un espace libre (9) cylindrique permettant l'oscillation du tube (5, 11, 15) se trouve au moins partiellement entre la face extérieure du tube (5, 11, 15) et la face intérieure de l'ouverture de passage (4).

3. Implant ou cathéter selon la revendication 1 ou 2, en ce que l'épaisseur de paroi du tube (5, 11, 15) est comprise entre 200 et 600 µm, de préférence entre 300 et 500 µm et en particulier de manière privilégiée est d'environ 400 µm.

4. Implant ou cathéter selon l'une des revendications précédentes, en ce que le diamètre intérieur de la lumière (6) du tube (5, 11, 15) est compris entre 2,5 et 3,5 mm, de préférence est égal à environ 3,0 mm.

5. Implant ou cathéter selon l'une des revendications précédentes, en ce que la partie du tube (5, 11 ,15) pouvant être mise en vibration est une partie centrale, dont l'épaisseur de paroi est réduite par rapport aux parties d'extrémité du tube (5, 11, 15).

6. Implant ou cathéter selon l'une des revendications précédentes, en ce que le tube (5, 11, 15) est fixé à une position supérieure (12) et à une position inférieure (13) dans l'implant (1, 10, 14) ou le cathéter et la longueur du tube (5, 11, 15) est supérieure à la distance entre la position supérieure (12) et la position inférieure (13).

7. Implant ou cathéter selon l'une des revendications précédentes, en ce que le tube (5, 11, 15) est formé de telle sorte que les parties (17) présentant un diamètre élargi alternent avec des parties (16) présentant un diamètre réduit.

8. Implant ou cathéter selon l'une des revendications précédentes, en ce que le tube (5, 11, 15) est réalisé à partir d'un élastomère, de préférence à partir d'un matériau silicone.
